# EUROPÄISCHE PATENTANMELDUNG

(11) **EP 1 212 994 A2**
(43) Veröffentlichungstag der Anmeldung: **12.06.2002**
(21) Anmeldenummer: 01129543.3
(22) Anmeldetag: 11.12.2001
(51) Int. Cl.: A61F 2/46

(54) **Gerät zum mechanischen Abtragen von harten Ablagerungen aus dem Lumen von weicheren Knochenschäften**

(30) Priorität: 11.12.2000 DE 20020944 U; 23.04.2001 DE 10119388
(71) Anmelder: Ort, Uwe, Dr., 97450 Arnstein (DE)
(72) Erfinder: Ort, Uwe, Dr., 97450 Arnstein (DE)
(74) Vertreter: HOFFMANN - EITLE

(57) **Zusammenfassung**

Um das rückstandsfreie Entfernen von harten Ablagerungen aus dem Lumen von weicheren Knochenschäften unter Minimieren von Knochenschädigungen zu ermöglichen, wird ein Gerät vorgeschlagen, das umfasst: ein Gehäuse (1, 2), ein mit dem Gehäuse verbundenes, eine Längsachse aufweisendes Werkzeug (26) und eine mit dem Gehäuse verbundene, eine Längsachse aufweisende Werkzeugführung (32, 33, 34); das Werkzeug (26) ist zumindest über einen Bereich seiner Länge mit einer Profilierung (36) versehen, die das Abtragen der Ablagerungen ermöglicht; das Werkzeug (26) und/oder die Werkzeugführung (32, 33, 34) sind/ist über eine Lagerung (14, 15, 16) derart beweglich an dem Gehäuse (1, 2) angebracht, dass sie quer zu ihren Längsachsen eine Relativbewegung von einer nahen zu einer entfernten Lage ihrer Längsachsen ausführen können; und die Werkzeugführung weist einen distalen Abschnitt (33) auf, der in der achsnahen Lage quer zu ihrer Längsrichtung über den profilierten Längenbereich des Werkzeugs vorsteht und die Führung des Werkzeugs (26) relativ zum Knochenschaft ermöglicht.

## Beschreibung

### TECHNISCHES GEBIET

Die vorliegende Erfindung betrifft ein Gerät zum mechanischen Abtragen von harten Ablagerungen aus dem Lumen von weicheren Knochenschäften, insbesondere ein Gerät zum mechanischen Abtragen von Palacos (Knochenzement) aus dem Lumen des Oberschenkelschaftes.

Derartige Geräte kommen insbesondere dann zur Anwendung, wenn bei menschlichen oder tierischen Patienten ein Hüftumbau notwendig wird, beispielsweise ein Umbau von einem vorhandenen Implantat auf ein neues Implantat.

Bei einem derartigen Hüftumbau muss der Knochenzement (Palacos), der das zu ersetzende Implantat in dem Knochen gehalten hat, möglichst restlos aus dem Knochenschaft entfernt werden, um zu gewährleisten, dass der Knochen das neue Implantat akzeptiert und mit diesem sicher verwächst. Selbst geringe Rückstände in Hohlräumen oder Hinterschneidungen des Knochenschaftes können das sichere Verwachsen mit dem neuen Implantat gefährden. Eine besondere Schwierigkeit ergibt sich dadurch, dass der Palacos im Vergleich zum Knochenschaft härter ist.

### STAND DER TECHNIK

Bislang ist es übliche Praxis, dass ein derartiger Hüftumbau von Hand durchgeführt wird. Bekannte Methoden für einen manuellen Hüftumbau umfassen auch die sogenannte Weber-Methode.

Diese Methode beruht darauf, dass der zu behandelnde Knochenschaft an seinem das Implantat enthaltenden Ende entlang seiner Längsachse ungefähr mittig aufgeschnitten wird. Im Anschluss an den Schnitt werden die sich durch den Schnitt ergebenden Hälften aufgeklappt, dann gereinigt und anschließend wieder mit Bändern zusammengefügt. Die bekannte Weber-Methode ist somit maximal invasiv. Der Reinigungsvorgang wird üblicherweise mit Hammer und Meißel durchgeführt. Die bei der manuellen Abtragung des intraluminaren Palacos im Oberschenkel verwendeten Meißel gibt es in unterschiedlichen Ausführungen bezüglich ihrer Breite, Länge und Krümmung. Eine derartige Reinigung ist jedoch nicht nur zu ungenau, sondern ebenfalls nicht wiederholbar. Überdies kann es zu ungewünschten Knochenschädigungen kommen.

Aus der DE 196 24 446 C1 ist ein chirurgisches Instrument zum mechanischen Entfernen von Knochenzement bekannt, das auf dem Entfernen des Knochenzements über Stoßwellen beruht. Bei dem bekanten Gerät besteht ein Kolbenelement aus einem Projektil, das auf eine hohe Endgeschwindigkeit beschleunigt werden kann. Das Projektilkolbenelement induziert eine Stoßwelle in das aus einer Sonde bestehende Meißelwerkzeug. Die Sondenspitze überträgt die Stoßwelle auf den Knochenzement. Das bekannte Gerät wird endoskopartig in den weitgehend intakten Knochenschaft eingeführt, nachdem aus diesem zuvor das Implantat entfernt wurde. Die Sondenspitze wird dann freihändig an den Knochenzementresten vorbeigeführt, um diese abzutragen.

Das bekannte Gerät weist jedoch den Nachteil auf, dass die Stoßwellen eine vergleichsweise geringe Eindringtiefe entfalten, und somit den Palacos beispielsweise aus in dem Knochenschaft gebildeten Hinterschneidungen in der Grenzschicht zwischen Palacos und Knochen nicht entfernen können.

Des weiteren ist es ebenfalls bekannt, den intraluminaren Palacos über Ultraschall oder dergleichen zu erweichen und damit die Möglichkeit der manuellen Entfernung des Palacos zu vereinfachen. Dieses Verfahren ist allerdings nur auf thermoplastische Palacos-Werkstoffe anwendbar.

Schließlich offenbart die DE 198 59 412 A1 ein chirurgisches Instrument zum Entfernen von Zement aus einer Knochenmarkhöhle beim Ersatz von Endoprothesen, allerdings nur zum Entfernen von distalen Zementresten. Zu den proximalen peripheren Zementresten wird in der DE 198 59 412 A1 angegeben, dass sie in herkömmlicher Weise mit Meißeln entfernt werden können.

### DARSTELLUNG DER ERFINDUNG

Der vorliegenden Erfindung liegt das technische Problem zugrunde, das nahezu rückstandsfreie Entfernen von harten Ablagerungen aus dem Lumen von weicheren Knochenschäften unter Minimieren von Knochenschädigungen zu ermöglichen.

Diese Aufgabe wird durch ein Gerät mit den Merkmalen von Anspruch 1 gelöst. Vorteilhafte Weiterbildungen sind in den Unteransprüchen angegeben.

Erfindungsgemäß werden die harten Ablagerungen aus den vergleichsweise weicheren Knochenschäften mittels der Profilierung des Werkzeugs abgetragen. Um das Werkzeug relativ zum Knochenschaft - und somit ebenfalls relativ zu den zu entfernenden Ablagerungen - zu führen, ist eine Werkzeugführung vorgesehen. Werkzeug und/oder Werkzeugführung sind/ist derart beweglich, dass ihre Längsachsen sich relativ zueinender bewegen können, voneinander weg zu einer achsentfernten Lage und aufeinander zu zu einer achsnahen Lage. Typischerweise fallen die Längsachsen in der achsnahen Lage zusammen. Die Werkzeugführung weist einen distalen Abschnitt auf, der in der achsnahen Lage quer zur Längsrichtung der Werkzeugführung vorsteht, über den profilierten Längenbereich des Werkzeugs. Auf diese Weise ist nicht nur eine sichere Führung des Geräts relativ zum Knochenschaft sichergestellt, indem beispielsweise der distale Abschnitt der Werkzeugführung im Knochenschaft verankert wird, sondern es kann ebenfalls der profilierte Längenbereich des Werkzeugs in der achsnahen Lage keine unbeabsichtigten Knochenschädigungen ausführen, da der distale Abschnitt der Werkzeugführung über ihn vorsteht. Auch in der achsentfernten Lage sind unbeabsichtigte Knochenschädigungen unterbunden, denn eine Führung des Werkzeugs ist über beispielsweise die Verankerung des distalen Abschnitts im Knochenschaft gewährleistet. Zugleich kann auch aus Hinterschneidungen, Hohlräumen und ähnlichem des Knochengewebes jede harte Ablagerung nahezu rückstandslos entfernt werden, denn über die sichere Führung des Werkzeugs können solche Stellen gezielt erreicht werden, ohne umliegendes Knochengewebe übermäßig nachteilig zu beeinflussen.

Es ist bevorzugt, dass die Werkzeugführung eine im Querschnitt zumindest einseitig offene Form aufweist, die bei einer Bewegung von der achsentfernten zur achsnahen Lage einen Eintritt des Werkzeugs in die Werkzeugführung ermöglicht. Auf diese Weise bilden Werkzeugführung und Werkzeug, die beide typischerweise eine lange schlanke Form aufweisen, eine mechanische Einheit, so dass das Gerät bei der Handhabung weniger wahrscheinlich beschädigt werden kann. Insbesondere wird auf diese Weise das Werkzeug auch bei einer Handhabung des Gerätes außerhalb von Knochenschäften vor Beschädigungen geschützt.

Um die Führung des Werkzeugs über die Werkzeugführung zu ermöglichen, ist nach einer Weiterbildung der Erfindung vorgesehen, dass das Werkzeug in der achsentfernten Lage teilweise in der Werkzeugführung eingetreten bleibt. Die Werkzeugführung übernimmt somit eine doppelte Führungsfunktion, einerseits die Führung des Werkzeugs relativ zum Knochenschaft, andererseits die Führung des Werkzeugs relativ zur Werkzeugführung. Es wird somit die Verbindung von Werkzeug und Gehäuse vereinfacht, denn die Anforderungen an ihre mechanische Festigkeit können gesenkt werden.

Diese Ausbildungen werden besonders erleichtert, wenn die Werkzeugführung einen längserstreckten Rundstab und das Werkzeug ein flaches Raspelblatt umfassen. Der längserstreckte Rundstab gemäß dieser Weiterbildung der Erfindung weist eine hohe inhärente Festigkeit gegen Biegen und Knicken auf, und eine Raspel hat sich zum mechanischen Abtragen von Ablagerungen als besonders wirkungsvoll bewährt.

Die erfindungsgemäß vorgesehene Relativbewegung zwischen Werkzeug und Werkzeugführung wird gemäß einer bevorzugten Ausführungsform der vorliegenden Erfindung dadurch erzielt, dass das Werkzeug mit dem Gehäuse quer zu seiner Längsachse unbeweglich verbunden ist, und die Werkzeugführung mit dem Gehäuse derart verbunden ist, dass sie quer zur Längsachse des Werkzeugs beweglich ist. Dies ermöglicht es, eine gegebenenfalls vorgesehene Bewegung des Werkzeugs zum Abtragen der Ablagerungen von der Relativbewegung zwischen Werkzeug und Werkzeugführung zu entkoppeln, wodurch die zum Ermöglichen dieser beiden Bewegungen erforderlichen Konstruktionen weniger aufwendig sind.

Eine gemäß der zuletzt genannten Weiterbildung vorgesehene bewegliche Verbindung der Werkzeugführung mit dem Gehäuse wird dann auf besonders stabile Art und Weise erzielt, wenn die Verbindung eine Lagerung mit in einem Parallelogramm angeordneten Führungsgabeln aufweist. Diese Parallelogrammanordnung ermöglicht es bei geeigneter Wahl der Länge der Parallelogrammseiten, der Werkzeugführung einen gewünschten Freiheitsgrad der Bewegung zu verleihen, zugleich aber unerwünschte Bewegungen in anderen Freiheitsgraden weitestgehend zu unterbinden.

Um das erfindungsgemäße Gerät möglichst einhändig bedienen zu können, ist gemäß einer weiteren Fortbildung der Erfindung vorgesehen, dass das Werkzeug und die Werkzeugführung über einen an dem Gehäuse vorgesehenen Griff zwischen der achsnahen und der achsentfernten Lage bewegbar sind. Typischerweise wird der Griff an einer der im Parallelogramm angeordneten Führungsgabeln befestigt sein, was zusätzliche Bauteile unnötig macht und zugleich einer Einhandbedienung nicht im Wege steht.

Das erfindungsgemäße Gerät ist dann besonders vielseitig einsetzbar, wenn der distale Abschnitt - was gemäß einer Weiterbildung der Erfindung vorgesehen ist - lösbar mit der verbleibenden Werkzeugführung verbunden ist. Auf diese Weise kann der distale Abschnitt entsprechend der Form und/oder Größe des zu behandelnden Lumens gewählt werden, was dem Einsatz in unterschiedlichen Körperbereichen oder bei unterschiedlich gebauten Patienten entspricht.

Gemäß der vorliegenden Erfindung ist ferner vorgesehen, dass der distale Abschnitt die Form eines Kugelsegments aufweist. Diese Ausführungsform ist deswegen besonders bevorzugt, weil eine Kugelform das Einführen des distalen Abschnitts in das zu behandelnde Lumen dann besonders gut unterstützt, wenn der Durchmesser des Lumens im wesentlichen dem Durchmesser des distalen Abschnitts entspricht. Im eingeführten Zustand weisen der distale Abschnitt und das Lumen dann eine ringförmige Berührung auf, was es ermöglicht, dass das gesamte Gerät mit der Längsachse von Werkzeugführung und Werkzeug um den distalen Abschnitt geschwenkt werden kann, und dennoch relativ zum Knochenschaft sicher geführt wird.

Da es erwünscht sein kann, über die Profilierung Ablagerungen in einer Bewegung abzutragen, die nicht manuell erzeugt wird, kann die vorliegende Erfindung einen Antrieb für das Werkzeug aufweisen. Es ist besonders bevorzugt, dass dieser Antrieb über Energie gespeist wird, der im typischen Einsatzgebiet des erfindungsgemäßen Geräts ohne weiteres zur Verfügung steht. Dies ist beispielsweise in einer Krankenhausumgebung dann verwirklicht, wenn der Antrieb für das Werkzeug ein Druckluftantrieb ist.

Die Abtragung der Ablagerungen wird bei einem längserstreckten Werkzeug geeigneter Weise in einer Hub-/Senkbewegung ausgeführt. Besonders wirksam ist die Abtragung dann, wenn gemäß einer weiteren Fortbildung der Erfindung sowohl der Hub- als auch der Senkanteil dieser Bewegung angetrieben sind.

Eine zugleich platzsparende aber dennoch zuverlässige und stabile Lösung für den Antrieb der Hub-/Senkbewegung ist der einer weiteren Fortbildung der Erfindung entsprechende Kurbeltrieb. Um einen schnelleren Werkzeugwechsel, einen leichteren Transport des Gerätes, und/oder eine einfachere Sterilisierung von Geräteteilen zu vereinfachen, ist darüber hinaus bevorzugt, wenn das Werkzeug lösbar mit dem Kurbeltrieb verbunden ist.

Schutz wird ebenfalls beansprucht für eine Kombination aus einem solchen Gerät mit einer Vielzahl von distalen, in der achsnahen Lage quer vorstehenden Abschnitten, die sich in ihren Formen und/oder Größen unterscheiden. Auf diese Weise kann dem Benutzer des Geräts ein Satz zur Verfügung gestellt werden, der ohne weiteres in den unterschiedlichsten Lumen verwendbar ist. Der Benutzer muss dann lediglich für den jeweiligen Einsatz den geeigneten distalen Abschnitt auswählen, ihn mit der verbleibenden Werkzeugführung verbinden, wodurch das erfindungsgemäße Gerät einsatzbereit ist.

### KURZE BESCHREIBUNG DER ZEICHNUNGEN

Die vorliegende Erfindung wird nun unter Bezugnahme auf die beigefügten Figuren ausführlicher beschrieben und erläutert. Es zeigt:
- Fig. 1: eine teilweise aufgebrochene Seitenansicht des erfindungsgemäßen Geräts gemäß einer ersten Ausführungsform;
- Fig. 2: eine der Fig. 1 entsprechende Untersicht;
- Fig. 3: eine der Fig. 1 entsprechende Draufsicht;
- Fig. 4: die in Fig. 1 mit IV gekennzeichnete Einzelheit;
- Fig. 5: die in Fig. 3 mit V gekennzeichnete Einzelheit; und
- Fig. 6a bis 6d: verschiedene Lagen von Werkzeug und Werkzeugführung des erfindungsgemäßen Geräts relativ zueinander, von einer zweiten Ausführungsform des erfindungsgemäßen Geräts.

### AUSFÜHRLICHE BESCHREIBUNG BEVORZUGTER AUSFÜHRUNGSFORMEN

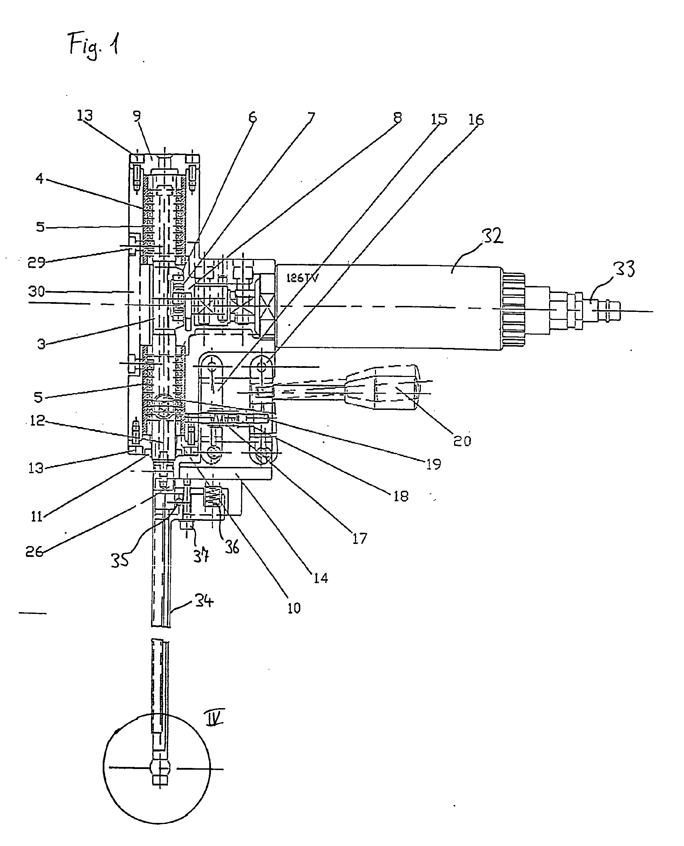

Zunächst unter Bezugnahme auf die Fig. 1 ist mit 3 eine Führungsstange bezeichnet, die Teil des Kurbeltriebs für das lösbar mit der Führungsstange verbundene Werkzeug 26 bildet. Die lösbare Verbindung wird bevorzugt über eine Schraubverbindung (Maschinenschraube 12) hergestellt. Die Führungsstange 3 ist in einer Führungsbüchse 4 des Gehäuses über Kugeln 5 axial derart beweglich geführt, dass sie sich entlang ihrer Längsachse bewegen und um die Längsachse drehen kann, weiter aber keine Freiheitsgrade in der Bewegung aufweist.

An der Führungsstange 3 ist ein ringförmiger Vorsprung vorgesehen, in dem ein Gleitstein 6 sitzt. Der Gleitstein 6 ist z.B. über ein Pendelkugellager 7 mit einer Kurbelwelle 8 verbunden. Die Kurbelwelle 8 wiederum wird über einen herkömmlichen Druckluftmotor 32 angetrieben, der über eine Standardkupplung 33 mit einer Druckluftquelle verbunden werden kann. Bei Betätigen des Druckluftmotors 32 über einen (nicht dargestellten) Schalter beginnt die Kurbelwelle sich zu drehen, wobei ein Zapfen der Kurbelwelle wie dargestellt exzentrisch zur Drehachse der Kurbelwelle über das Pendelkugellager 7 und den Gleitstein 6 die Führungsstange 3 zu einer Hub/Senkbewegung veranlasst, bei der sowohl der Hubanteil der Bewegung, als auch der Senkanteil der Bewegung angetrieben ist.

Bevorzugt ist die Führungsstange 3 hohlgebohrt, so dass die Maschinenschraube 12 zur lösbaren Befestigung des Werkzeugs 26 mit der Führungsstange 3 von der dem Werkzeug entfernten Seite von außen durch das Gehäuse zugänglich ist. Werkzeugwechsel können auf diese Weise schnell und einfach durchgeführt werden, indem die Maschinenschraube 12 von der werkzeugentfernten Seite der Führungsstange aus gelöst wird, woraufhin das Werkzeug 26 ohne Weiteres von der Führungsstange 3 abgenommen werden kann. Um sicherzustellen, dass die Maschinenschraube 12 zum Werkzeugwechsel zugänglich ist, kann beispielsweise der in Fig. 1 mit 9 bezeichnete obere Gehäusedeckel ebenfalls eine entsprechende Bohrung aufweisen. Der untere Gehäusedeckel 10 weist eine entsprechend größere Bohrung auf, durch die die Führungsstange 3 durchtreten kann. Er ist ferner mit einem Abstreifer 11 versehen, der sicherstellt, dass von außen keine Verschmutzungen in den Antrieb des Werkzeugs eindringen können, und Schmier- und Betriebsstoffe aus dem Werkzeug nicht in die Knochenhöhle gelangen. Der obere Gehäusedeckel 9 und der untere Gehäusedeckel 10 sind über Maschinenschrauben 13 an dem Gehäuse befestigt. Das Gehäuse ist im Allgemeinen zweiteilig und umfasst einen Gehäuseoberteil 1 und einen Gehäuseunterteil 2.

Um den Antrieb für Wartungsarbeiten oder ähnliches zugänglich zu machen, ist ein über Maschinenschrauben 29 an dem Gehäuse befestigter Führungsdeckel 30 vorgesehen.

In Fig. 1 ist des weiteren ein Griff 20 dargestellt, der an der wie folgt aufgebauten beweglichen Lagerung zum Erzeugen einer Relativbewegung zwischen Werkzeug 26 und Werkzeugführung 34 befestigt ist.

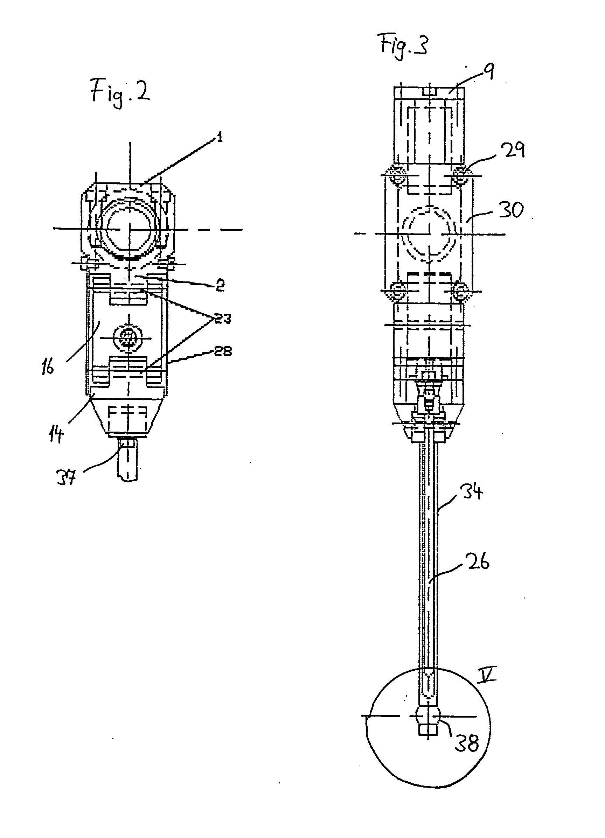

Der Griff 20 wird an einer ersten Führungsgabel 16 befestigt, typischerweise angeschraubt, wie auch aus Fig. 2 hervorgeht. Die Führungsgabel 16 nimmt in dem Raum zwischen ihren Zinken auf der einen Seite einen Vorsprung des Gehäuses, auf der anderen Seite einen Vorsprung einer Platte 14 der Werkzeugführung auf. Die Führungsgabel 16 ist über Zylinderstifte 23 drehbeweglich an diesen beiden Vorsprüngen befestigt. Parallel zur Führungsgabel 16 ist eine weitere Führungsgabel 15 (Fig. 1) auf ähnliche Weise an dem Gehäusevorsprung und an dem Vorsprung der Werkzeugführungsplatte 14 angebracht. Über einen Gewindestift 19, der über eine an einer Mutter anliegenden Druckfeder 17 vorgespannt ist, werden die Führungsgabeln 15 und 16 in der nicht betätigten Position des Griffs 20 zu der gewünschten Ruhestellung vorgespannt, die weiter unten unter Bezugnahme auf die Fig. 6a bis 6d beschrieben ist.

Die Werkzeugführung 34 ist über einen Stift 35 drehbeweglich an der Platte 14 befestigt. Die Drehbewegung um den Stift 35 findet gegen die Vorspannung einer Feder 36 statt und wird von einer Stellschraube 37 begrenzt. Über die Stellschraube 37 kann somit beispielsweise der Maximalwinkel eingestellt werden, den der längserstreckte Werkzeugführungsstab 34 relativ zur Platte 14 einnehmen kann.

Der Werkzeugführungsstab 34 weist distal, d.h. gehäusefern, einen quer zu seiner Längserstreckung vorstehenden Abschnitt 38 auf, der unter Bezugnahme auf die Fig. 4 und 5 erläutert ist.

In Fig. 4 ist der distale Endbereich des Werkzeugs 26 zu sehen, insbesondere dessen Profilierung 39. Bevorzugt erstreckt sich die Profilierung 39 des Werkzeugs 26 über die gesamte Länge des Werkzeugs, mit Ausnahme der Befestigung des Werkzeugs am proximalen Endbereich am Führungsstab 3. Es ist jedoch ebenfalls möglich, die Profilierung 39 auf einen ausgewählten Längenbereich des Werkzeugs 26 zu beschränken. Es hat sich für das Abtragen von Palacos aus Knochenschäften als besonders geeignet herausgestellt, wenn die Profilierung 39 als Raspel ausgeführt ist.

In der Fig. 4 ist weiterhin der distale Endbereich des Werkzeugführungsstabs 34 dargestellt. Aus einer Gegenüberstellung der Fig. 4 und 5 geht besonders gut hervor, dass der Werkzeugführungsstab 34 gemäß der bevorzugten Ausführungsform einen annähernd kreisrunden Querschnitt aufweist, der über eine Aussparung 40 einseitig offen ist. Die Aussparung 40 ermöglicht den Eintritt und Austritt des Werkzeugs 26. Im eingetretenen Zustand ist das Werkzeug vollkommen, d.h. inklusive der Profilierung, in dem Werkzeugführungsstab 34 aufgenommen. Es ist jedoch ebenfalls denkbar, den Führungsstab anders auszuführen, z.B. derart flach, dass er an dem Werkzeug 26 an der der Profilierung 39 gegenüberliegenden Seite vorbei läuft.

Der Werkzeugführungsstab 34 weist einen distalen Abschnitt 38 auf, der in der Richtung quer zur Längsachse 41 des Werkzeugführungsstabs 34 vorsteht. Der distale Abschnitt 38 ist bevorzugt kugel- oder kugelsegmentförmig ausgebildet, wie auch in den Fig. 4 und 5 dargestellt. Um den distalen Abschnitt 38 von dem Führungsstab 34 lösen und wieder an ihm befestigen zu können, ist auf der distalen Stirnseite eine nicht näher erläuterte Verschraubung vorgesehen. Auf diese Weise ist es möglich, verschiedene distale Abschnitte 38 bereitzustellen, die in ihrer Größe und/oder Form dem zu behandelnden Knochenlumen entsprechen. Die distalen Abschnitte 38 selbst sind dann bevorzugt starr ausgeführt. Dies entspricht der derzeit bevorzugten Ausführungsform.

Es ist jedoch ebenfalls möglich, wie insbesondere aus Fig. 5 hervorgeht, in dem Führungsstab 34 eine Längsbohrung 42 zum Versorgen des distalen Abschnitts 38 mit Druckluft vorzusehen. Über ein weiteres Bohrungssystem (Fig. 5) kann die von proximal zugeführte Druckluft über die Längsbohrung 42 und weitere Bohrungen in den distalen Abschnitt 38 eintreten. Sofern der distale Abschnitt 38 flexibel ausgeführt ist, kann er ohne weiteres in das Lumen des zu behandelnden Knochenschaftes eingeführt werden, und wird sich erst bei Beaufschlagen mit Druckluft ausdehnen und fest an die Lumenwandung anlegen. Somit wäre eine sichere Verankerung ermöglicht.

In den Fig. 6a bis 6d ist das zuvor beschriebene Gerät in einer abgewandelten Ausführungsform dargestellt.

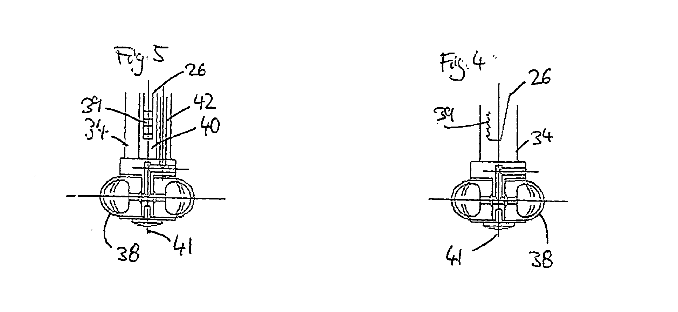

Die Ausführungsform des Geräts in den Fig. 6a bis 6d unterscheidet sich von der in den Fig. 1 bis 5 dargestellten dadurch, dass bei ihr zur Erzeugung der Relativbewegung zwischen Werkzeug 26 und Werkzeugführung 34 über den Griff 20 das Werkzeug 26 relativ zum Gehäuse bewegt wird. Die Werkzeugführung 34 verbleibt bei Betätigen des Griffes 20 relativ zum Gehäuse unverändert. Eine zur Auslenkung des angetriebenen Werkzeugs 26 geeignete Einrichtung wird dem Fachwissen des Durchschnittsfachmanns unterstellt. Anregungen hierzu gibt die in den Fig. 1 bis 5 dargestellte erste Ausführungsform. Bei der Ausführungsform der Fig. 6a bis 6d ist es beispielsweise denkbar, Werkzeug samt Druckluftantrieb relativ zum Gerätegehäuse zu verschwenken, was die Mechanik des Antriebs der Hub/Senkbewegung des Werkzeugs 26 vereinfacht.

Die zuvor beschriebenen Geräte können wie folgt gehandhabt werden. Rein beispielhaft ist dies anhand der Fig. 6a bis 6d beschrieben.

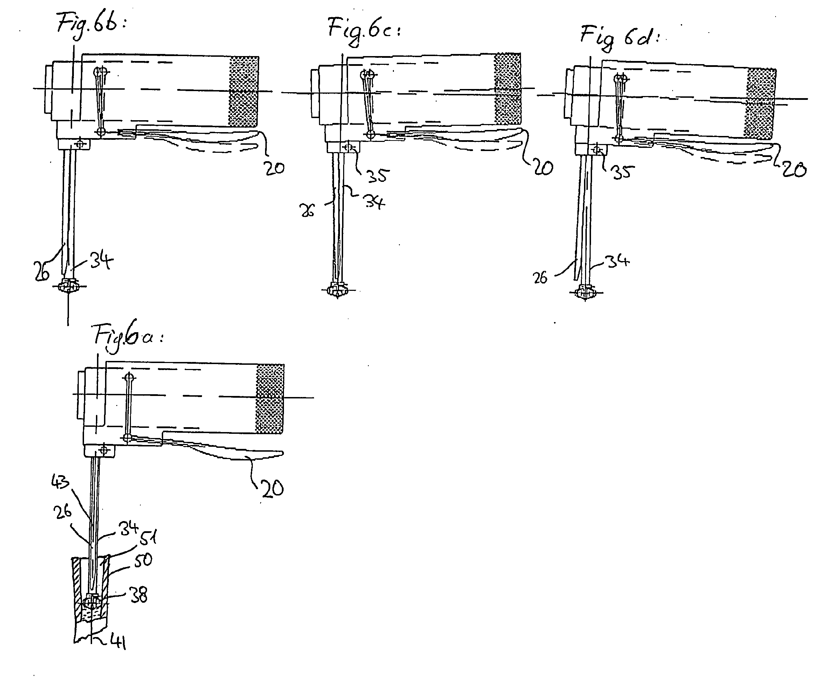

Insbesondere aus der Fig. 6a geht hervor, wie das Gerät in das Lumen 51 eines Knochenschaftes 50 eingesetzt und dort mit dem distalen Abschnitt 38 der Werkzeugführung 34 verankert werden kann. In den Fig. 6b, 6c und 6d ist zur Vereinfachung der Darstellung der Knochenschaft weggelassen worden.

In Fig. 6a ist das Werkzeug 26 mit seiner Längsachse 43 nahe zur Längsachse 41 der Werkzeugführung 34. Die Achsen fallen zusammen. Dies ist die Lage, in die Werkzeug und/oder Werkzeugführung typischerweise vorgespannt sind, d.h. es ist die Nulllage des Griffs 20. Das Werkzeug wird unter leichtem Druck auf den Boden des Lumens 51 des Knochenschafts 50 eingebracht, bis der distale Abschnitt 38 an der Innenwand des Knochenschafts 50 anliegt. Die hierbei entstehende Berührung ist eine Linienberührung in Form eines Rings, was in der Fig. 6a schematisch über die Horizontalachse des distalen Abschnitts 38 angedeutet ist. Der distale Abschnitt 38 ist entweder aus einer Auswahl verschiedener distaler Abschnitte entsprechend dem Innendurchmesser des Knochenschaftes in Bodennähe des Lumens 51 ausgewählt, oder der distale Abschnitt 38 kann gemäß der Ausführungsform der Fig. 4 und 5 mit Druckluft beaufschlagt werden, um sich an die Innenwandung des Knochenschafts 50 anzulegen. Durch diese Berührung ist gewährleistet, dass die Werkzeugführung 34 und somit ebenfalls das Werkzeug 26 nicht unbeabsichtigt verrutscht.

Ausgehend von der in Fig. 6a dargestellten Lage ist es beispielsweise denkbar - durch Betätigen des Griffes 20 in Richtung des Gehäuses (Fig. 6b) - das Werkzeug 26 in eine Lage zu bringen, in der seine Längsachse von der Längsachse der Werkzeugführung 34 entfernt ist. In Fig. 6b ist dargestellt, dass in der achsentfernten Lage die Längsachsen von Werkzeug 26 und Werkzeugführung 34 nach wie vor parallel sind. Zudem ist der Figur entnehmbar, dass das Werkzeug 26 teilweise in der Werkzeugführung 34 eingetreten verbleibt, wodurch eine Führung des Werkzeugs sichergestellt und die mechanische Belastung des Werkzeugantriebs verringert ist.

Von dieser Lage ausgehend, kann über Verschwenken des Gehäuses mit Werkzeug 26 relativ zur Werkzeugführung 34 die achsparallele Lage verlassen werden (Fig. 6c). Dies kann beispielsweise durch Drehung der Werkzeugführung 34 mit gezogenem Griff 20 um den Stift 35 ermöglicht werden.

Ebenfalls ist es denkbar, die relative Lage der Längsachsen des Werkzeugs 26 und der Werkzeugführung 34 in die andere Richtung von der parallelen Lage abweichen zu lassen (Fig. 6d). Dies kann erreicht werden, indem das Gehäuse mit gezogenem Griff 20 in die andere Richtung geschwenkt wird.

Durch diese Abweichung von der in Fig. 6a gezeigten Lage, in der die Werkzeugführung 34 die Profilierung des Werkzeugs 26 umgibt, so dass ein mechanisches Abtragen von Ablagerungen im Lumen 51 des Knochenschaftes 50 nicht möglich ist, wird die gezielte lokale Abtragung über eine spanende Bearbeitung möglich. Hierbei ist über die Verankerung der Werkzeugführung mittels des distalen Abschnitts 38 im Lumen 51 gleichzeitig unterbunden, dass das Werkzeug 26 verrutscht und es zu unbeabsichtigten Schädigungen des Knochenschaftes 50 kommt.

Diese maschinelle Entfernung von beispielsweise intraluminärem Palacos im Oberschenkelschaft kann durch Raspeln verschiedener Größe erzielt werden. Ein besonders bevorzugtes Ausführungsbeispiel sieht vor, dass das Werkzeug ein sowohl hub- als auch senkangetriebener einseitig mit einer Sägezahnprofilierung versehener, etwa 20 cm langer Metallstab ist. Der Metallstab weist eine Breite von 3 x 3 mm auf. Er ist in die Ausnehmung eines etwa 22 cm langen Rundstabs als Werkzeugführung 34 eingebettet. Der Längenunterschied ermöglicht einen Hub des Werkzeugs von annähernd 2 cm. Die Raspel kann dann durch Kippen am proximalen Anteil und am distalen Anteil der Werkzeugführung 34 durch manuell dosierte Kraft ohne Abweichung sicher im Lumen, z.B. im Oberschenkellumen geführt werden. Weiterhin ist es möglich, die Raspel 26 auf ganzer Länge bis zur zweifachen Eigentiefe dosiert aus dem Rundstab der Werkzeugführung 34 zu hebeln, ohne dass es zu unkontrollierten Abweichungen kommt.

Der distale Abschnitt 38, der zur Verankerung des Gerätes relativ zum Knochenschaft dient, kann über eine abnehmbare, in verschiedenen Größen vorhandene Kugel am distalen Ende der Werkzeugführung vorgesehen werden. Die Kugel ist in ihrem Durchmesser auf den intraluminaren Durchmesser abgestimmt, um eine gesicherte Verankerung des gesamten Gerätes bereitzustellen. Wie in den Fig. 6a bis 6d gezeigt ist, kann des weiteren bei Hub und Schub das Gerät im Lumen des Oberschenkelschaftes dosiert im ganzen gekippt werden, ohne dass die Raspel unkontrolliert abweicht und das umliegende knöcherne Gewebe zerstört.

Als bevorzugte Werkstoffe sind für den Antrieb des Werkzeugs Aluminium und V2A-Stahl vorgesehen. Das Gehäuse besteht zur einfachen Sterilisierung aus Nirosta-Stahl. Die weiteren Antriebsteile bestehen geeigneter Weise ebenfalls aus V2A-Stahl. Werkzeugführung 34 und distaler Abschnitt 38 werden typischerweise ebenfalls aus V2A-Stahl bestehen. Obwohl dies aufgrund der leichteren Verfügbarkeit und der einfacheren Bearbeitung derzeit die bevorzugten Werkstoffe sind, ist ebenfalls daran gedacht, diese Bauteile in biokompatiblen Werkstoffen auszuführen.

Für die Raspel hat es sich besonders bewährt, sie in Titan auszuführen. Andere für Werkzeuge für spanende Bearbeitung geeignete Werkstoffe sind jedoch ebenfalls einsetzbar.

## Patentansprüche

1. Gerät zum mechanischen Abtragen von harten Ablagerungen aus dem Lumen von weicheren Knochenschäften, insbesondere von Palacos aus dem Lumen des Oberschenkelschaftes, umfassend:
- ein Gehäuse (1, 2), ein mit dem Gehäuse verbundenes, eine Längsachse aufweisendes Werkzeug (26) und eine mit dem Gehäuse verbundene, eine Längsachse aufweisende Werkzeugführung (32, 33, 34);
- das Werkzeug (26) ist zumindest über einen Bereich seiner Länge mit einer Profilierung (36) versehen, die das Abtragen der Ablagerungen ermöglicht;
- das Werkzeug (26) und/oder die Werkzeugführung (32, 33, 34) sind/ist über eine Lagerung (14, 15, 16) derart beweglich an dem Gehäuse (1, 2) angebracht, dass sie quer zu ihren Längsachsen eine Relativbewegung von einer nahen zu einer entfernten Lage ihrer Längsachsen ausführen können; und
- die Werkzeugführung weist einen distalen Abschnitt (33) auf, der in der achsnahen Lage quer zu ihrer Längsrichtung über den profilierten Längenbereich des Werkzeugs vorsteht und die Führung des Werkzeugs (26) relativ zum Knochenschaft ermöglicht.

2. Gerät nach Anspruch 1, **dadurch gekennzeichnet, dass** die Werkzeugführung (32, 33, 34) eine im Querschnitt zumindest einseitig offene Form aufweist, die bei einer Bewegung von der achsentfernten zur achsnahen Lage einen Eintritt des Werkzeugs (26) in die Werkzeugführung ermöglicht.

3. Gerät nach Anspruch 2, **dadurch gekennzeichnet, dass** das Werkzeug (26) in der achsentfernten Lage teilweise in der Werkzeugführung (32, 33, 34) eingetreten verbleibt, so dass eine Führung des Werkzeugs relativ zur Werkzeugführung bereitgestellt ist.

4. Gerät nach einem der vorhergehenden Ansprüche, **dadurch gekennzeichnet, dass** die Werkzeugführung einen längserstreckten Rundstab (32) und das Werkzeug ein flaches Raspelblatt (26) umfasst.

5. Gerät nach einem der vorhergehenden Ansprüche, **dadurch gekennzeichnet, dass** das Werkzeug (26) mit dem Gehäuse (1, 2) quer zu seiner Längsachse unbeweglich, und die Werkzeugführung (32, 33, 34) mit dem Gehäuse (1, 2) quer zur Längsachse des Werkzeugs (26) beweglich verbunden ist.

6. Gerät nach Anspruch 5, **dadurch gekennzeichnet, dass** die Verbindung der Werkzeugführung (32, 33, 34) eine Lagerung mit in einem Parallelogramm angeordneten Führungsgabeln (15, 16) aufweist.

7. Gerät nach einem der vorhergehenden Ansprüche, **dadurch gekennzeichnet, dass** das Werkzeug (26) und die Werkzeugführung (32, 33, 34) über einen an dem Gehäuse vorgesehenen Griff (20) zwischen der achsnahen und der achsentfernten Lage bewegbar sind.

8. Gerät nach den Ansprüchen 6 und 7, **dadurch gekennzeichnet, dass** der Griff (20) an einer der im Parallelogramm angeordneten Führungsgabeln (16) befestigt ist.

9. Gerät nach einem der vorhergehenden Ansprüche, **dadurch gekennzeichnet, dass** der distale Abschnitt (33) lösbar mit der verbleibenden Werkzeugführung (32) verbunden ist, so dass er entsprechend der Form und/oder Größe des Lumens wählbar ist.

10. Gerät nach einem der vorhergehenden Ansprüche, **dadurch gekennzeichnet, dass** der distale Abschnitt (33) die Form eines Kugelsegments aufweist.

11. Gerät nach einem der vorhergehenden Ansprüche, **dadurch gekennzeichnet, dass** es einen Antrieb für das Werkzeug (26) aufweist, bevorzugt einen Druckluftantrieb.

12. Gerät nach Anspruch 11, **dadurch gekennzeichnet, dass** das Werkzeug (26) im Betrieb des Geräts eine reine Hub-/Senkbewegung ausführt, wobei bevorzugt sowohl der Hub- als auch der Senkanteil dieser Bewegung angetrieben sind.

13. Gerät nach Anspruch 12, **dadurch gekennzeichnet, dass** der Antrieb der Hub-/Senkbewegung einen mit dem Werkzeugantrieb gekoppelten Kurbeltrieb (3, 4, 5, 6, 7, 8) umfasst.

14. Gerät nach Anspruch 13, **dadurch gekennzeichnet, dass** das Werkzeug (26) lösbar mit dem Kurbeltrieb verbunden ist.

15. Kombination aus einem Gerät nach mindestens einem der vorhergehenden Ansprüche mit einer Vielzahl von distalen, in der achsnahen Lage quer vorstehenden Abschnitten (33), die sich in ihren Formen und/oder Größen unterscheiden.
